Europäisches Patentamt

**European Patent Office**

Office européen des brevets

(11) Veröffentlichungsnummer: **0 209 711 B1**

(12) **EUROPÄISCHE PATENTSCHRIFT**

(45) Veröffentlichungstag der Patentschrift: **13.03.91**

(21) Anmeldenummer: **86108031.5**

(22) Anmeldetag: **12.06.86**

(51) Int. Cl.⁵: **G01N 33/49**, G01N 15/00

(54) Messeinrichtung zum Messen des Verformungsvermögens von roten Blutkörperchen.

(30) Priorität: **21.06.85 DE 3522186**

(43) Veröffentlichungstag der Anmeldung:
**28.01.87 Patentblatt 87/05**

(45) Bekanntmachung des Hinweises auf die
Patenterteilung:
**13.03.91 Patentblatt 91/11**

(84) Benannte Vertragsstaaten:
**AT BE CH DE FR GB IT LI LU NL SE**

(56) Entgegenhaltungen:
**DE-A- 2 750 447      DE-A- 2 824 831**
**DE-A- 3 215 719      GB-A- 1 476 210**
**US-A- 3 815 022      US-A- 4 428 669**

(73) Patentinhaber: **HOECHST AKTIENGESELL-SCHAFT**
**Postfach 80 03 20**
**W-6230 Frankfurt am Main 80(DE)**

(72) Erfinder: **Jahn, Helmut**
**Ludwig-Marx-Strasse 24**
**W-6500 Mainz(DE)**
Erfinder: **Karger, Waldemar**
**Memelstrasse 18**
**W-6233 Kelkheim (Taunus)(DE)**

Rank Xerox (UK) Business Services

**Beschreibung**

Gegenstand der Erfindung ist eine Meßeinrichtung zum Messen des Verformungsvermögens von roten Blutkörperchen, Erythrozyten, bestehend aus einer Meßkammer, die durch eine Folie in zwei Kammerräume unterteilt ist, wobei die Folie eine Durchströmöffnung aufweist, deren Durchmesser kleiner als der Ruhedurchmesser eines roten Blutkörperchens ist, die Kammerräume so gestaltet sind, daß ein Druckgefälle zwischen den beiden Kammerräumen und damit eine Strömung von einem zum anderen Kammerraum zustande kommt, und beidseits der Folie im Bereich der Öffnung wenigstens je eine Elektrode angeordnet ist, die mit einer Wechselspannungsquelle verbunden ist.

Eine Meßeinrichtung der genannten Art ist nach der Deutschen Patentschrift DE-A-32 15 719 bekannt. Zum Messen des Verformungsvermögens der Erythrozyten wird der eine Kammerraum der Meßkammer mit einer Mischung aus Pufferlösung und Vollblut und der andere mit Pufferlösung gefüllt. Da auf Grund des Kanalverlaufs ein hydrostatisches Druckgefälle vorhanden ist, werden die Erythrozyten zum Passieren durch die Öffnung in der Folie veranlaßt. Als Maß für die Verformbarkeit gilt dabei die Zeit, die die einzelnen Erythrozyten benötigen, um eine Einzellochmembran zu passieren. Bei der Passage des Erythrozyten durch die Öffnung der Folie ändert sich der elektrische Gesamtwiderstand der Meßkammer. Diese Änderung wird registriert; sie ist ein direktes Maß für die Passagezeit. An die auf beiden Seiten der Öffnung befindlichen Elektroden wird eine Wechselspannung hochohmig angelegt und der während der Passage des Erythrozyten durch die Öffnung sich ändernde Gesamtwiderstand der Meßzelle als Spannungsänderung erfaßt.

Nachteilig bei der Wechselspannungsmessung ist, daß die Folie selbst einen Kondensator darstellt, so daß dem Ohm'schen Widerstand der Öffnung noch ein parasitärer kapazitiver Widerstand parallel geschaltet ist. Dazu kommen noch parasitäre Shuntkapazitäten. Bei steigender Frequenz erhält man zwar eine steigende Auflösung der Passagezeiten, es sinkt jedoch der Wert des kapazitiven Widerstandes. Daraus folgt eine bei steigenden Meßfrequenzen immer kleiner werdende Änderung des Meßsignals beim Durchtritt der Erythrozyten durch die Öffnung in der Folie, so daß eine aufwendige Auswerteelektronik erforderlich wird.

Hier will die Erfindung Abhilfe schaffen. Aufgabe der Erfindung ist es demnach, eine meßeinrichtung zum Messen des Verformungsvermögens von roten Blutkörperchen zu schaffen, bei der ein um etwa eine Größenordnung größeres Meßsignal bei gegebener Wechselspannungsfrequenz erhältlich ist.

Die Aufgabe wird dadurch gelöst, daß in der Verbindung einer Elektrode mit der Wechselstromquelle eine Strommeßeinrichtung angeordnet ist.

In Ausgestaltung der Erfindung ist eine der Elektroden massenah mit der Wechselstromquelle verbunden und in dieser Verbindung die Strommeßeinrichtung angeordnet. Als Strommeßeinrichtung kann ein als invertierender Verstärker arbeitender Operationsverstärker mit nachgeschaltetem Analog/Digital-Wandler mit der Elektrode verbunden sein. Dem Analog/Digital-Wandler kann ein Filter und/oder ein Spitzenwertdetektor, z.B. ein Zweiwegdetektor, vorgeschaltet sein.

Bei sorgfältiger Minimierung der Ballastkapazitäten in der Meßzelle und den elektrischen Anschlüssen kann mit der erfindungsgemäßen Meßeinrichtung ein um etwa eine Größenordnung höheres Meßsignal erreicht werden. Dadurch sind neben der Ermittlung der Passagezeit auch erstmals Aussagen über den qualitativen Kurvenverlauf des elektrischen Meßsignals während der Passage des roten Blutkörperchens durch die Folie möglich.

Die Erfindung wird im folgenden anhand der Figuren näher erläutert. Es zeigen

Figur 1    die elektrische Schaltung der Meßeinrichtung,

Figur 2    einen Teil der elektrischen Schaltung der Meßkammer mit alternativer Anordnung des Operationsverstärkers,

Figur 3    das digitalisierte Eingangssignal für den Mikroprozessor.

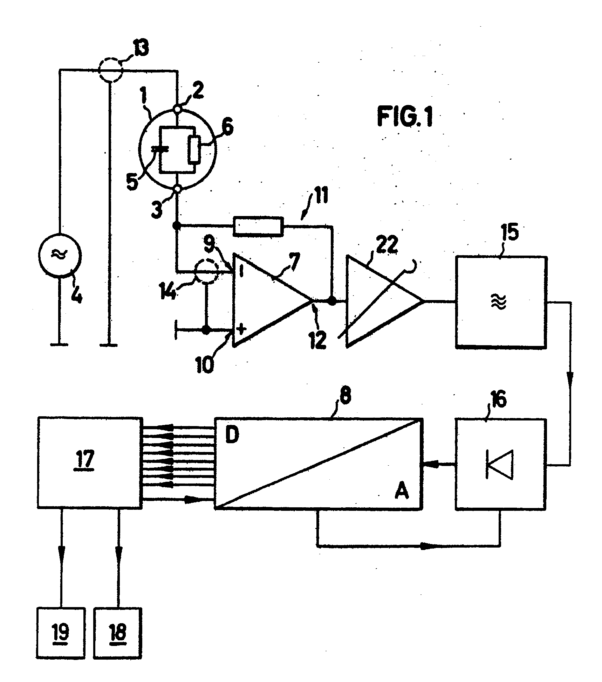

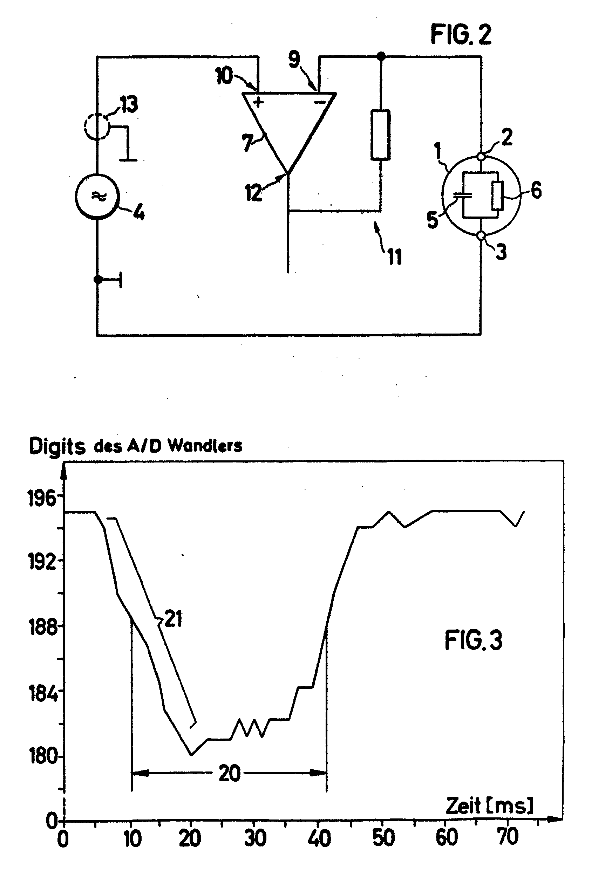

Die Meßkammer der Meßeinrichtung wie sie beispielsweise in der Deutschen Patentschrift DE-A-32 15 719 beschrieben ist, ist durch eine Folie in zwei Kammerräume unterteilt. Die Folie weist eine Durchströmöffnung auf, deren Durchmesser kleiner als der Ruhedurchmesser eines roten Blutkörperchens ist. Die Kammerräume sind so gestaltet, daß ein Druckgefälle zwischen den beiden Kammerräumen und damit eine Strömung von einem zum anderen Kammerraum zustande kommt, wenn sich in den Kammerräumen eine die roten Blutkörperchen enthaltende Flüssigkeit befindet. Beiderseits der Folie ist im Bereich der Durchströmöffnung wenigstens je eine Elektrode angeordnet, die mit einer Wechselspannungsquelle 4 verbunden ist. In den Figuren 1 und 2 ist die Meßkammer 1 als elektrisches Ersatzschaltbild dargestellt. Dabei stehen die Anschlüsse 2 und 3 für die Elektroden, der unvermeidbare kapazitive Widerstand 5 für die Folie und der Ohm'sche Widerstand 6 für den konduktiven Verbindungspfad zwischen den Elektroden durch die Öffnung der Folie. Zum Erfassen des Widerstandes 5/6 und seiner Änderung wird der elektrische Strom direkt gemessen, der im Meßkreis Wechselstromquelle 4, Meßkammer 1 fließt. Dies kann prinzipiell an jedem Ort im Meßkreis geschehen. In der Verbindung zwischen einer der Elektroden 2, 3 mit der Wechselstromquelle 4 ist eine Strommeßeinrichtung angeordnet. Die Strommeßeinrichtung kann aus einem als invertierender Verstärker arbeitenden Operationsverstärker 7 bestehen. Dabei ist der invertierende Eingang 9 des Verstärkers mit der Elektrode 2 bzw. 3 verbunden, während der nicht invertierende Eingang 10 mit der Wechselspannungsquelle 4 bzw. mit Masse verbunden ist. Vorzugsweise wird der Strom am kalten Leitungsende der Meßkammer gemessen. Der invertierende Eingang 9 des Operationsverstärkers 7 ist mit der Elektrode 3 verbunden und spiegelt sich ein fiktives Massepotential durch Rückkopplung 11 eines gleichgroßen, entgegen gesetzten Stroms vor. Hierdurch wird eine exakt stromproportionale Ausgangsspannung am Ausgang 12 des Operationsverstärkers 7 erzeugt. Ein Spannungsabfall zwischen der Elektrode 3 und Masse tritt bei der Strommessung praktisch nicht auf. Durch die besondere Schaltungstechnik ist auch jede sonst schädliche Verbindung der Meßkammer 1 mit Masse unerheblich. Es treten prinzipiell keine parasitären Ströme im Meßpfad auf, weshalb auch auf den Einsatz eines teuren Instrumentationsverstärkers verzichtet werden kann. Somit können als Zuleitung auch längere, abgeschirmte Leitungen 13, 14 unbedenklich eingesetzt werden. Bedingt durch die Niederohmigkeit, sowohl am "heißen" Einspeiseende (Elektrode 2) als auch am "kalten" Masseende (Elektrode 3) der Meßkammer 1 werden Störeinstrahlungen, auch im Spektrum der Nutzfrequenz, weitgehend unterdrückt. Gegebenenfalls nach Unterdrückung weiterer Störungen durch aktive Filter 15 kann mit einem Spitzenwertdetektor 16 eine Spitzenwertdetektion vorgenommen werden Das Signal wird anschließend zur weiteren Auswertung einem Analog/Digitalwandler 8 zugeführt, der eine ausreichende Auflösung besitzt, um eine anschließende Amplitudenbewertung zu ermöglichen. Der Spitzenwertdetektor 16 sorgt dafür, daß dem Analog/Digitalwandler 8 eine ausreichende Signalkonstanz während der Wandlungszeit zur Verfügung steht. In manchen Fällen kann die Verwendung eines Zweiwegdetektors günstiger sein. Durch Einsatz eines dem Analog/ Digitalwandler 8 folgenden Mikroprozessors 17 ist es möglich, ohne eine Änderung des bestehenden Hardware-Aufbaus verschiedene Auswertungen vorzunehmen, die die Information medizinischer Relevanz aus den Meßdaten ermittelt. Über Auswerte-Algorithmen kann eine langsam driftende Änderung des Meßkammerstromes kompensiert und kürzere Störimpulse eliminiert werden, die nicht dem charakteristischen Verlauf eines Erythrozytendurchtritts entsprechen. Gegebenenfalls kann dem Operationsverstärker ein einstellbarer Verstärker 22 nachgeschaltet werden.

Es wird schließlich der totale Zeitbedarf 20 (Figur 3) für die einzelne Passage eines Erythrozyten durch die Folie ermittelt, hierüber eine Statistik errechnet und die Ergebnisse im Display 18 angezeigt und/oder mittels Drucker 19 ausgedruckt. Ebenfalls wird die detaillierte Erfassung besonders wichtiger Phasen der Passagen ermöglicht, so z.B. der Verlauf der Elongationsphase 21 (Figur 3) der Erythrozyten beim Eintritt in die Öffnung der Folie.

## Ansprüche

1. Meßeinrichtung zum Messen des Verformungsvermögens von roten Blutkörperchen bestehend aus einer Meßkammer, die durch eine Folie in zwei Kammerräume unterteilt ist, wobei die Folie eine Durchströmöffnung aufweist, deren Durchmesser kleiner als der Ruhedurchmesser eines roten Blutkörperchens ist, die Kammerräume so gestaltet sind, daß ein statisches Druckgefälle zwischen den beiden Kammerräumen und damit eine strömung von einem zum anderen Kammerraum zustande kommt, und beiderseits der Folie im Bereich der Durchströmöffnung wenigstens je eine Elektrode angeordnet ist, die mit einer Wechselspannungsquelle verbunden ist, dadurch gekennzeichnet, daß eine der Elektroden massenah mit der Wechselspannungsquelle verbunden ist, und in dieser Verbindung eine Strommeßeinrichtung angeordnet ist.

2. Meßeinrichtung nach Anspruch 1, dadurch gekennzeichnet, daß als Strommeßeinrichtung ein als invertierender Verstärker arbeitender Operationsverstärker (7) mit nachgeschaltetem Analog/Digital-Wandler (8) mit der Elektrode verbunden ist.

3. Meßeinrichtung nach Anspruch 1, dadurch gekennzeichnet, daß zwischen Operationsverstärker (7) und Analog/Digital-Wandler (8), ein Filter (15) angeordnet ist.

4. Meßeinrichtung nach Anspruch 2, dadurch gekennzeichnet, daß dem Analog/Digital-Wandler (8) ein Spitzenwertdetektor (16) vorgeschaltet ist.

5. Meßeinrichtung nach Anspruch 4, dadurch gekennzeichnet, daß als Spitzenwertdetektor ein Zweiwegdetektor verwendet wird.

## Claims

1. A measuring device for the measurement of the deformability of red blood corpuscles, which device comprises a measurement chamber, which is subdivided by a foil into two chamber spaces, the foil having a passage opening, the diameter of which is smaller than the diameter, at rest, of a red corpuscle, the chamber spaces being formed in such a manner that a static pressure gradient comes into existence between the two chamber spaces and thus a flow takes place from one chamber space to the other, and at least one electrode being disposed on each of the two sides of the foil in the region of the passage opening, each respective electrode being connected to an alternating voltage source, wherein one of the electrodes is connected to the alternating voltage source close to ground, and a current-measuring device is disposed in this connection.

2. The measuring device as claimed in claim 1, wherein as current-measuring device an operational amplifier (7) operating as an inverting amplifier, with an analog/digital converter (8) connected behind the same, is connected to the electrode.

3. The measuring device as claimed in olaim 1, wherein a filter (15) is disposed between the operational amplifier (7) and the analog/digital converter (8).

4. The measuring device as claimed in claim 2, wherein a peak value detector (16) is connected before the analog/digital converter (8).

5. The measuring device as claimed in claim 4, wherein a full-wave detector is employed as peak value detector.

## Revendications

1. Dispositif de mesure servant à mesurer la capacité de déformation de globules rouges, constitué par une chambre de mesure, qui est subdivisée par une feuille en deux espaces, et dans lequel la feuille possède une ouverture de passage dont le diamètre est inférieur au diamètre au repos d'un globule rouge, les espaces séparés dans la chambre sont agencés de telle sorte qu'il s'établit un gradient statique de pression entre les deux espaces de la chambre et par conséquent un écoulement d'un espace en direction de l'autre espace de la chambre, et dans lequel au moins respectivement une électrode, qui est reliée à une source de tension alternative, est disposée des deux côtés de la feuille au voisinage de l'ouverture de passage, caractérisé en ce que l'une des électrodes est reliée, à proximité de la masse, à la source de tension alternative et qu'un dispositif ampèremétrique est disposé dans cette liaison.

2. Dispositif de mesure selon la revendication 1, caractérisé en ce qu'un amplificateur opérationnel (7) travaillant en tant qu'amplificateur inverseur et en aval duquel est branche un convertisseur analogique/numérique (8), est relié en tant que dispositif ampèremétrique à l'électrode.

3. Dispositif de mesure selon la revendication 1, caractérisé en ce qu'un filtre (15) est monté entre l'amplificateur opérationnel (7) et le convertisseur analogique/numérique (8).

4. Dispositif de mesure selon la revendication 2, caractérisé en ce qu'un détecteur de valeurs maximales (13) est branché en amont du convertisseur analogique/numérique (8).

5. Dispositif de mesure selon la revendication 4, caractérisé en ce qu'un détecteur bidirectionnel est utilisé en tant que détecteur de valeurs maximales.

FIG.1

FIG. 2

Digits des A/D Wandlers

FIG. 3

Zeit [ms]